# EUROPEAN PATENT APPLICATION

(11) **EP 4 667 498 A1**
(43) Date of publication of application: **24.12.2025**
(21) Application number: 24756047.7
(22) Date of filing: 04.02.2024
(51) Int. Cl.: C07K 19/00, A61K 38/26, A61P 3/10

(54) **GLP-1 ANALOG-FC FUSION PROTEIN AND USE THEREOF**

(30) Priority: 15.02.2023 CN 202310114868
(71) Applicant: Zhang, Jing, Gusu District Suzhou, Jiangsu 215000 (CN)
(72) Inventor: Zhang, Jing, Gusu District Suzhou, Jiangsu 215000 (CN)
(74) Representative: Schiweck Weinzierl Koch Patentanwälte Partnerschaft mbB
(86) International application number: PCT/CN2024/075676
(87) International publication number: WO 2024/169673

(57) **Abstract**

Provided is a GLP-1 analog-Fc fusion protein, comprising a GLP-1 analog, a peptide linker and the Fc portion of an immunoglobulin, wherein the GLP-1 analog is linked to the Fc portion of the immunoglobulin via a peptide linker. The amino acid sequence of the GLP-1 analog-Fc fusion protein is shown in SEQ ID NO. 7. The GLP-1 analog-Fc fusion protein shows no side effect of severe gastrointestinal tract aversive reactions in use, and has more significant effects on lowering blood glucose and inhibiting food intake.

## Description

### TECHNICAL FIELD

The invention relates to the field of biotechnology, specifically to a fusion protein of a GLP-1 analogue and Fc, as well as preparation method thereof and uses thereof.

### BACKGROUND

Glucagon-like peptide-1 (GLP-1) is a brain-gut peptide secreted by ileal endocrine cells, initially isolated and extracted from the intestinal mucosa. It is one of the main components of insulinotropic hormones. The initially produced GLP-1 is a 37-amino acid peptide that is inactive. After 6 N-terminal amino acids are removed by enzymatic cleavage, it becomes the biologically active GLP-1 (7-37) (SEQ ID NO. 1).

GLP-1 can promote insulin secretion by pancreatic β-cells and reduce glucagon secretion by pancreatic α-cells, thereby lowering blood glucose. The action of GLP-1 depends on its receptor, the glucagon-like peptide-1 receptor (GLP-1R), making GLP-1R an important target for diabetes treatment. Glucagon-like peptide-1 receptor agonists (GLP-1RAs) are novel hypoglycemic drugs launched in recent years. Due to advantages such as strong hypoglycemic effect, low hypoglycemia risk, good weight loss effect, and cardiovascular benefits, the emergence of GLP-1RAs has revolutionized the entire diabetes treatment market.

Native GLP-1 is rapidly degraded by dipeptidyl peptidase-4 (DPP-4) in the body, with a half-life as short as 1.5-2 minutes, rendering it therapeutically impractical. Therefore, the primary challenge in developing GLP-1RAs is to extend their half-life. Current industry strategies include: altering the amino acids at specific positions in GLP-1 to reduce DPP-4 mediated clearance, e.g., Exenatide; employing fatty acid side chains on a GLP-1RA molecule that bind to serum albumin, minimizing renal glomerular filtration and further extending the half-life, e.g., Liraglutide and Semaglutide; creating a fusion protein by conjugating the C-terminus of a GLP-1RA to an Fc fragment of immunoglobulin to extend the half-life, e.g., Dulaglutide; and the like. Among these, forming a "GLP-1RA-Fc" fusion protein of a GLP-1RA with an Fc fragment of immunoglobulin offers more advantages, for example, enabling a prolonged plasma half-life; forming a stable dimer via disulfide bonds in the Fc hinge region, with the Fc domain capable of independent folding to ensure stability of the partner molecule both in vivo and in vitro; and facilitating simplified purification of the fusion protein through adsorption to Protein A affinity columns, etc.

However, currently marketed GLP-1RAs all have serious gastrointestinal side effects, including nausea, vomiting, diarrhea, and abdominal distension. These side effects lead to dose limitations, resulting in current doses of GLP-1RAs being far from achieving saturation binding to their receptors for maximum efficacy. Simultaneously, in the current administration of GLP-1RA drugs, it is common for patients to discontinue treatment prematurely, require concomitant antiemetic medication or even develop depression, due to intolerance of these side effects. Therefore, there remains a need in the art to develop novel GLP-1-based drugs, especially new GLP-1RAs which have stronger biological activity and longer duration of action, but do not induce nausea and vomiting, thereby further optimizing clinical effects such as hypoglycemia and weight loss and improving patient compliance.

### SUMMARY OF THE INVENTION

An object of the present disclosure is to provide a fusion protein formed by a GLP-1 analogue and the Fc fragment of an immunoglobulin. In the fusion protein, the employed GLP-1 analogue should have the maximum sequence identity (i.e., homology) with native GLP-1 to minimize potential immunogenicity from exogenous sequences; at the same time, through fusion with the Fc fragment, the fusion protein should overcome the defects of GLP-1-based drugs in the art that are prone to cause vomiting and other related side effects. Another object of the present disclosure is to provide the preparation method and uses of the fusion protein.

The present disclosure provides technical solutions as follows.

In a first aspect, the present disclosure provides a GLP-1 analogue-Fc fusion protein, comprising a GLP-1 analogue, a peptide linker, and an immunoglobulin Fc portion, in which the GLP-1 analogue is linked to the immunoglobulin Fc portion via the peptide linker.

In the GLP-1 analogue-Fc fusion protein provided by the present disclosure, the GLP-1 analogue comprises the amino acid sequence shown in SEQ ID NO. 2:
HGEGTFPSDVSSYLEGQAAKEFIAWLVKGRG (SEQ ID NO. 2).

Compared to the amino acid sequence of native GLP-1 (7-37) shown below, the amino acid sequence of the GLP-1 analogue provided by the present disclosure has Glycine (G) at position 2 (A2G) and Proline (P) at position 7 (T7P), thus exhibiting 94% sequence identity with the native GLP-1 (7-37):
HAEGTFTSDVSSYLEGQAAKEFIAWLVKGRG (SEQ ID NO. 1).

In the GLP-1 analogue-Fc fusion protein provided by the present disclosure, the immunoglobulin Fc portion can be the Fc portion of human IgG1, IgG2, IgG3, or IgG4. Preferably, the immunoglobulin Fc portion is the Fc portion of human IgG4. According to specific embodiments of the present disclosure, the immunoglobulin Fc portion comprises the amino acid sequence shown in SEQ ID NO. 3:

SKYGPPCPPCPAPEAAGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLG (SEQ ID NO. 3).

In the GLP-1 analogue-Fc fusion protein provided by the present disclosure, the peptide linker can be a peptide linker well-known in the art, such as a flexible peptide linker, as long as it does not adversely affect the functions of the GLP-1 analogue and the Fc portion within the fusion protein. Preferably, the peptide linker comprises a "GGGGS (SEQ ID NO. 4)" repeat sequence. According to specific embodiments of the present disclosure, the peptide linker comprises the amino acid sequence shown in SEQ ID NO. 5:
GGGGGGSGGGGSGGGGSAE (SEQ ID NO. 5).

Preferably, in the GLP-1 analogue-Fc fusion protein provided by the present disclosure, the C-terminus of the amino acid sequence of the GLP-1 analogue is linked via the peptide linker to the N-terminus of the amino acid sequence of the immunoglobulin Fc portion, i.e., the structure of the GLP-1 analogue-Fc fusion protein takes the form N'-GLP-1 analogue-linker-Fc-*C*'. According to specific embodiments of the present disclosure, the fusion protein comprises the amino acid sequence shown in SEQ ID NO. 6 or SEQ ID NO. 7, or the amino acid sequence of the fusion protein is shown in SEQ ID NO. 6 or SEQ ID NO. 7:

MDMRVPAQLLGLLLLWFPGSRCHGEGTFPSDVSSYLEGQAAKEFIAWLVKGRGGGGGGG
SGGGGSGGGGSAESKYGPPCPPCPAPEAAGGPSVFLFPPKPKDTLMISRTPEVTCVVVDVS

QEDPEVQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWESNGQP
ENNYKTTPPVLDSDGSFFLYSRLTVDKSRWQEGNVFSCSVMHEALHNHYTQKSLSLSLG
(SEQ ID NO. 6; underlined part is the signal peptide).

HGEGTFPSDVSSYLEGQAAKEFIAWLVKGRGGGGGGGSGGGGSGGGGSAESKYGPPCPP
CPAPEAAGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYSR
LTVDKSRWQEGNVFSCSVMHEALHNHYTQKSLSLSLG (SEQ ID NO. 7).

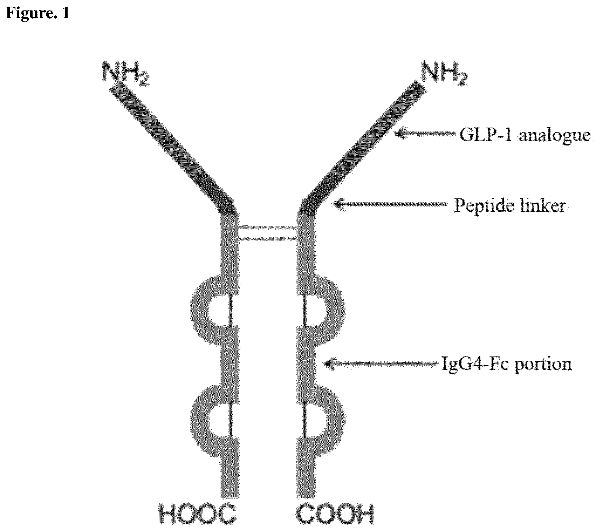

Preferably, the GLP-1 analogue-Fc fusion protein provided by the present disclosure exists in the form of a dimer, more preferably, in the form of a homodimer. According to specific embodiments of the present disclosure, the structure of the fusion protein is shown in Figure 1.

In a second aspect, the present disclosure provides a nucleic acid molecule, comprising a nucleotide sequence encoding the GLP-1 analogue-Fc fusion protein provided by the present disclosure. The nucleic acid molecule can be in DNA or RNA form; in which the DNA form includes cDNA, genomic DNA, or synthetic DNA; and the DNA can be single-stranded or doublestranded.

According to specific embodiments of the present disclosure, the nucleic acid molecule comprises the nucleotide sequence shown in SEQ ID NO. 8:

ATGGACATGCGCGTGCCCGCCCAGCTGCTGGGCCTGCTGCTGCTGTGGTTCCCCGGAT
CCCGATGCCATGGTGAAGGTACTTTTCCAAGTGATGTGAGCTCCTACCTGGAAGGCCA
GGCCGCTAAAGAGTTCATCGCTTGGCTCGTCAAGGGCAGAGGCGGAGGCGGAGGAGG
TGGCTCCGGCGGCGGCGGCTCTGGCGGCGGCGGATCTGCCGAGAGCAAGTACGGCCC
CCCATGTCCTCCTTGCCCTGCTCCTGAGGCCGCTGGCGGCCCTTCCGTGTTTCTGTTCC
CTCCCAAGCCAAAGGACACCCTGATGATCTCTCGGACCCCTGAAGTCACATGCGTGGT
GGTGGACGTGTCTCAAGAGGATCCCGAGGTGCAGTTTAACTGGTACGTGGATGGCGTG
GAAGTGCACAACGCCAAGACCAAGCCCAGAGAGGAACAGTTCAACTCCACCTACAGA
GTCGTTTCTGTGCTGACCGTGCTGCACCAGGACTGGCTGAACGGCAAAGAGTACAAGT
GCAAAGTGTCCAATAAGGGCCTGCCTAGCTCCATCGAGAAGACCATCTCCAAGGCCAA

GGGACAGCCTAGAGAACCTCAAGTGTACACACTGCCTCCTTCTCAGGAAGAGATGACC
AAGAACCAGGTGAGCCTGACCTGTCTGGTGAAGGGCTTCTACCCCTCCGACATCGCCG
TGGAATGGGAGTCTAATGGCCAGCCTGAGAACAACTACAAAACCACCCCACCTGTGCT
GGATTCTGACGGCTCTTTCTTCCTGTACAGCCGGCTGACCGTGGACAAGTCCCGGTGG
CAGGAGGGCAACGTGTTCTCCTGCTCCGTGATGCACGAGGCCCTGCATAACCACTATAC
ACAGAAGTCCCTGTCCCTGTCTTTGGGC (SEQ ID NO. 8).

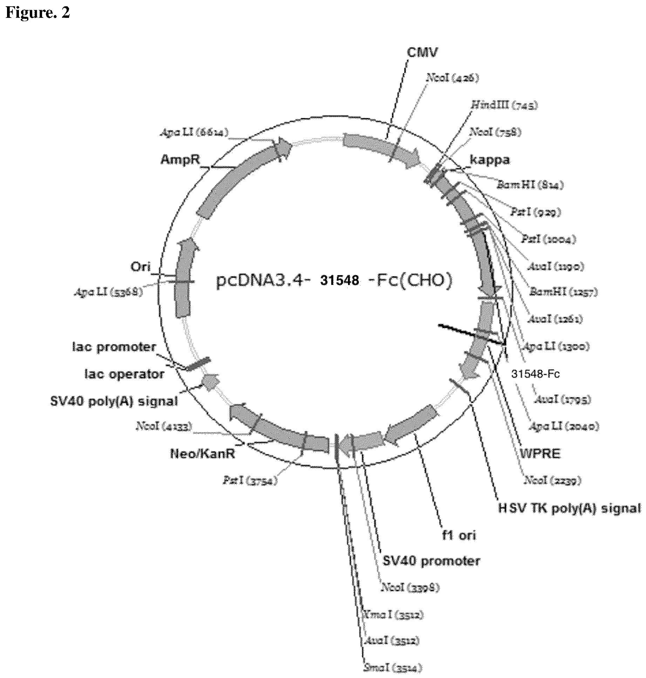

In a third aspect, the present disclosure provides a vector, comprising a nucleotide sequence encoding the GLP-1 analogue-Fc fusion protein provided by the present disclosure and/or comprising the nucleic acid molecule. Preferably, the vector is an expression vector. More preferably, the expression vector is a eukaryotic expression vector, such as a vector of pcDNA series. According to specific embodiments of the present disclosure, the structure of the expression vector is shown in Figure 2.

In a fourth aspect, the present disclosure provides a host cell, comprising a nucleotide sequence encoding the GLP-1 analogue-Fc fusion protein provided by the present disclosure, the nucleic acid molecule, and/or the vector, or transformed or transfected with a nucleotide sequence encoding the GLP-1 analogue-Fc fusion protein provided by the present disclosure, the nucleic acid molecule, and/or the vector. The host cell can be a prokaryotic cell, such as a bacterial cell, e.g., an E. coli cell; or a lower eukaryotic cell, such as a yeast cell, e.g., a a cell of Saccharomyces cerevisiae; or a higher eukaryotic cell, such as a mammalian cell. Preferably, the host cell is a mammalian cell. According to specific embodiments of the present disclosure, the host cell is a CHO cell.

In a fifth aspect, the present disclosure provides a method for producing the GLP-1 analogue-Fc fusion protein, comprising expressing the vector in a host cell.

In a sixth aspect, the present disclosure provides use of the GLP-1 analogue-Fc fusion protein in the manufacture of a medicament for treating type II diabetes, obesity, or overweight. Generally, "obesity" refers to a BMI (body mass index) no lower than 30, and "overweight" refers to a BMI higher than 25 but lower than 30.

In a seventh aspect, the present disclosure provides a pharmaceutical composition, comprising the GLP-1 analogue-Fc fusion protein and a pharmaceutically acceptable carrier. The pharmaceutically acceptable carrier can be a buffer, a stabilizer, an antioxidant, a preservative, a surfactant, a pH adjuster, etc., known to those skilled in the art which can be combined with functional proteins for preparing protein-based drugs. Preferably, the pharmaceutical composition is an injection, such as an intravenous injection or a subcutaneous injection.

In an eighth aspect, the present disclosure provides a method for treating diabetes, obesity, or overweight, comprising administering to a subject in need thereof the GLP-1 analogue-Fc fusion protein provided by the present disclosure itself or a pharmaceutical composition comprising the fusion protein. The subject is a mammal, preferably a primate, more preferably a human. The GLP-1 analogue-Fc fusion protein itself or the pharmaceutical composition comprising the fusion protein can be administered alone or in combination with other agents. Preferably, the GLP-1 analogue-Fc fusion protein itself or the pharmaceutical composition comprising the fusion protein is administered to the subject in need thereof by injection. More preferably, the GLP-1 analogue-Fc fusion protein itself or the pharmaceutical composition comprising the fusion protein is administered to the subject in need thereof by intravenous injection or subcutaneous injection.

Compared with prior arts, the present disclosure provides a fusion protein comprising a glucagon-like peptide-1 receptor agonist, the fusion protein being formed by fusion of a functional protein, namely a GLP-1 analogue, with an immunoglobulin Fc portion via a peptide linker.

Through structural modification of native GLP-1, specifically by amino acid substitutions at two positions in the native GLP-1, the inventors of the invention obtained a GLP-1 analogue with high identity (94%) to the native GLP-1. The amino acid substitutions enable the GLP-1 analogue exhibit a significantly improved molecular biological activity compared with the native GLP-1, while effectively circumvents potential immunogenicity attributable to exogenous sequences. Further, by fusing this GLP-1 analogue to the Fc portion of human IgG4 via a specific peptide linker, a GLP-1 analogue-Fc fusion protein was obtained.

Experiments demonstrated that, compared to several currently marketed GLP-1 receptor agonist hypoglycemic drugs, the GLP-1 analogue-Fc fusion protein provided by the present disclosure did not cause side effects such as nausea and vomiting during its administration; simultaneously, its effects on lowering blood glucose and inhibiting food intake were more significant, particularly as its food intake-inhibiting effect did not rely on adverse physiological reactions like nausea. Therefore, the use of the GLP-1 analogue-Fc fusion protein provided by the present disclosure can allow for a substantial increase in dose, thereby eliminating the need for dose titration and shortening the time to onset of effect. Additionally, by increasing the dose, the dosing interval can be extended, thereby reducing the number of injections required for patients.

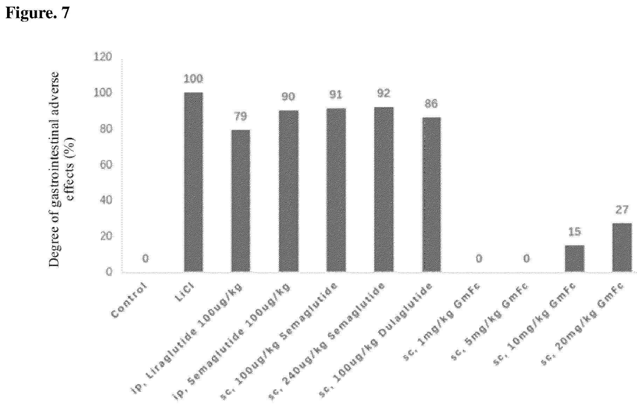

Specifically, extrapolating from the results shown in Figure 7 that a 20 mg/kg dose induced a degree of gastrointestinal adverse effects at 27%, a high dose of 60 mg/kg in a mouse (equivalent to a dose of 600 mg in a 60 kg human) will produce a degree of gastrointestinal adverse effects at 81%, which percentage remains significantly lower than those degrees of gastrointestinal adverse effects caused by the minimum injectable doses of currently marketed drugs: Semaglutide (1 mg per injection for a 60 kg human; 92%) and Dulaglutide (1 mg per injection for a 60 kg human; 86%). The injection doses in mice corresponding to the minimum injectable doses of Semaglutide (1 mg per injection for a 60 kg human) and Dulaglutide (1 mg per injection for a 60 kg human) were both 0.1 mg/kg. These results indicate that in clinic use, GmFc can be administered at a dose at least 100-fold higher on a per-weight basis than those of these two marketed drugs.

### BRIEF DESCRIPTION OF THE DRAWINGS

Embodiments of the invention are described in detail below with reference to the attached figures, in which:
Figure 1 shows an exemplary structure of the fusion protein provided by the present disclosure;
Figure 2 shows the map of pcDNA plasmid for expressing the fusion protein provided by the present disclosure in CHO cells;
Figure 3 shows the gel electrophoresis results of the fusion protein provided by the present disclosure;
Figure 4 shows the blood glucose-lowering effect of the fusion protein provided by the present disclosure;
Figure 5 shows the food intake-inhibiting effect of the fusion protein provided by the present disclosure;
Figure 6 illustrates the conditioned taste aversion (CTA) procedure; and
Figure 7 shows the results of gastrointestinal aversion effects of the fusion protein provided by the present disclosure.

### DETAILED DESCRIPTION OF PREFERRED EMBODIMENTS

The embodiments of the invention are illustrated below with reference to specific and concrete examples. It will be understood by those skilled in the art that these examples are merely illustrative of the invention and do not limit the scope of the invention in any way.

It should be understood that the terms used in the examples of the present disclosure are for describing the specific, concrete examples and are not intended to limit the scope of protection of the invention. Experimental methods without specific conditions indicated in the following examples are generally carried out under conventional conditions or according to the conditions recommended by the manufacturers.

Dulaglutide was purchased from Eli Lilly and Company. Liraglutide and Semaglutide were purchased from Novo Nordisk Inc.

### Example 1 Preparation of GLP-1 analogue-Fc fusion protein "GmFc"

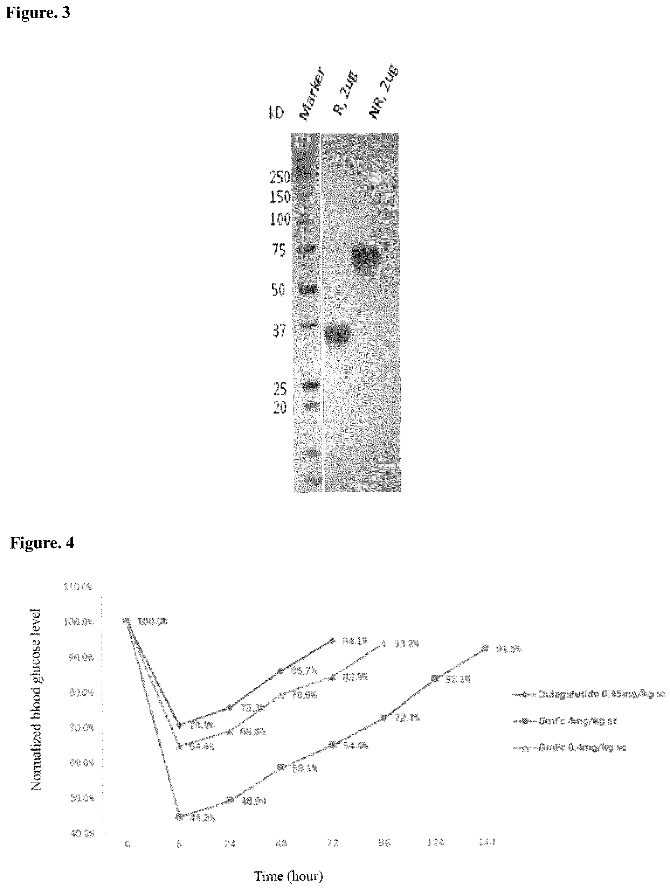

DNA (SEQ ID NO. 8) encoding the amino acid sequence of GmFc precursor (SEQ ID NO. 6) was chemically synthesized and then cloned into the expression plasmid pcDNA3.4-31548-Fc(CHO) (shown in Figure 2). Next, CHO cells were transiently transfected with the recombinant plasmid and after 9 days of cell culture and secretion expression, the cell-autonomously processed mature GmFc protein (SEQ ID NO. 7) was obtained from the culture broth. 1 liter of this culture broth was subjected to a two-step separation and purification process (the first step was protein A affinity chromatography using MabSelect Prism A available from Cytiva, USA, and the second step was size exclusion chromatography using Superdex 200 available from Cytiva, USA), ultimately yielding 36 mg of GmFc protein with purity greater than 98%, which was a homodimer with a molecular weight of 60 KD (Figure 3).

The transiently transfected cells were further screened and optimized, and a high expressing cell line stably producing GmFc was obtained for large-scale production.

### Example 2 Study on the blood glucose-lowering and food intake-inhibiting effects of the fusion protein GmFc

The blood glucose-lowering and food intake-inhibiting effects of the fusion protein GmFc were assessed in Streptozotocin (STZ)-induced diabetic mouse model.

Adult male C57BL/6J mice were used. After fasting for more than 12 hours, an intraperitoneal bolus injection of STZ solution at a dose of 45 mg/kg was administered to the mice. One week later, blood glucose levels were measured daily around 9:00 AM, and mice with blood glucose levels exceeding 20 mM were selected. These mice were randomly assigned into groups, 6 mice per group. The drug administration regimen for each group is shown in Table 1.

**Table 1. Drug administration in mice**

| | Drug | Dose | Route |
|---|---|---|---|
| GLP-1 receptor agonist hypoglycemic drug group | Dulaglutide | 0.45 mg/kg | S.C. |
| GmFc group | GmFc | 0.4 mg/kg | |
| GmFc group | GmFc | 4 mg/kg | |
| Negative control group | Physiological saline, equal volume | / | |
| No treatment group | / | / | |

Blood glucose levels were measured at 0 and 6 hours post-dosing on the day of drug administration. Animals were allowed free access to food and water. From day 2 onward, the total food consumption during the preceding night was recorded, and blood glucose levels were monitored at scheduled times dialy.

The results demonstrated that compared to the positive control, i.e., hypoglycemic drug Dulaglutide (0.45 mg/kg), both doses of GmFc (0.4 mg/kg and 4 mg/kg) effectively reduced blood glucose levels, with superior blood glucose-lowering efficacy to Dulaglutide (0.45 mg/kg). These outcomes are presented in Figure 4, where the Y-axis represents the percentage normalized blood glucose level to 22 mM (the blood glucose level of the animals before dosing) which was set at 100%, and the X-axis represents time post-administration in hours. The blood glucose levels of the mice in both the negative control group and no treatment group showed no any obvious fluctuation throughout the study period (data not shown).

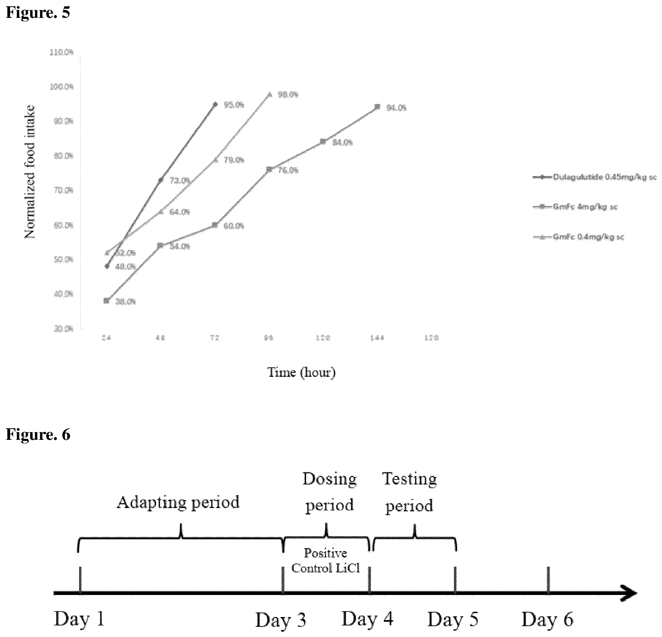

Furthermore, compared to the positive control, i.e., hypoglycemic drug Dulaglutide (0.45 mg/kg), both doses of GmFc (0.4 mg/kg and 4 mg/kg) significantly suppressed food intake, demonstrating superior food intake-inhibiting efficacy to Dulaglutide (0.45 mg/kg). These outcomes are presented in Figure 5, where the Y-axis represents the percentage normalized 24-hour food intake to the daily food intake of the no treatment animals which was set at 100%, and the X-axis represents time post-administration in hours. The daily food intake of the mice in the negative control group and no treatment group showed no any obvious fluctuation throughout the study period (data not shown).

These findings demonstrated that, the fusion protein GmFc effectively lowered blood glucose level and concurrently inhibited daily food intake in STZ-induced type 2 diabetic mice. Both the blood glucose-lowering and food intake-inhibiting effects of GmFc were shown to be significantly greater than those achieved by the positive control Dulaglutide at comparable doses.

### Example 3 Study on gastrointestinal adverse effects of the fusion protein GmFc

The gastrointestinal adverse effects of the fusion protein GmFc were assessed in conditioned taste aversion (CTA) mouse model.

Male C57BL/6J mice (20-25 g, 8-10 weeks old) were used. The mice were randomly assigned to groups, 10 mice per group. Days 1-3 were the adapting period; and on Day 4, the mice had scheduled water access for 30 minutes, and then were administered according to the regimens in Table 2. On Day 5, aversion index was measured to evaluate the severity of drug-induced gastrointestinal adverse effects (specific methodology detailed in the "CTA Procedure" section below).

The drug administration regimen for each group is shown in Table 2.

**Table 2. Drug administration in mice**

| | Drug | Dose | Route |
|---|---|---|---|
| Positive control drug | LiCl | 0.15 M, 40 mL/kg | i.p. |
| GLP-1 receptor agonist hypoglycemic drug group | Liraglutide | 0.1 mg/kg | i.p. |
| GLP-1 receptor agonist hypoglycemic drug group | Semaglutide | 0.1 mg/kg | i.p. |
| GLP-1 receptor agonist hypoglycemic drug group | Semaglutide | 0.1 mg/kg | s.c. |
| GLP-1 receptor agonist hypoglycemic drug group | Semaglutide | 0.24 mg/kg | s.c. |
| GLP-1 receptor agonist hypoglycemic drug group | Dulaglutide | 0.1 mg/kg | s.c. |
| Test article group | GmFc | 1.0 mg/kg | s.c. |
| Test article group | GmFc | 5.0 mg/kg | s.c. |
| Test article group | GmFc | 10 mg/kg | s.c. |
| Test article group | GmFc | 20 mg/kg | s.c. |
| Negative control group | Physiological saline | | s.c. |

### CTA Procedure:

When an animal first consumes a novel-tasting food and subsequently experiences visceral discomfort (e.g., nausea, vomiting, or abdominal pain), it forms a strong impression against consuming that food. Upon encountering food with the same taste again, the animal reduces or even rejects consuming it. A conditioned taste aversion (CTA) experiment can be established on this process. Generally, the novel taste is chosen based on the animal's preference, often a sweet liquid. Accordingly, for the learning and memory process, a 0.2% sodium saccharin solution was used as the conditioned stimulus (CS) in the experiment to leverage mice's inherent preference for sweetness, and the symptoms such as abdominal pain caused by intraperitoneal injection of lithium chloride (LiCl) were used as the unconditioned stimulus (US). By establishing an association between the CS and US, the aim to investigate taste-related memory in mice was achieved (Figure 6).
(1) Adapting period (Days 1-3): Mice were water-deprived for 24 hours prior to training (ad libitum access to chow maintained). Subsequently, for 3 consecutive days, mice underwent water training daily at fixed time: each mouse received tap water for 30 minutes. Water consumption was weighed and recorded, and a balanced drinking pattern at fixed time was established in the mice.
(2) Dosing period (Day 4): At fixed time, the adapted mice were given saccharin water (0.2% sodium saccharin solution) instead of tap water. After 30 minutes of drinking, the test sample or LiCl (0.15 M, 40 mL/kg, intraperitoneal injection) as the positive control was administered to induce abdominal discomfort in mice. Reduced activity, stretched posture, and occasional diarrhea might occur in the mice for a period afterward.
(3) Testing period (Day 5): If the mice experienced discomfort after drinking the saccharin water the previous day, they would form a short-term memory and develop an aversion to the drink, deliberately avoiding it the next day. So at specific time during testing, the mice were provided with two bottles, one containing tap water and the other saccharin water, and allowed to drink freely for 30 minutes. The consumption of tap water and saccharin water was separately weighed.

Aversion index (AI) was used to assess the degree of nausea induced by the test article in the animals: AI = (Amount of tap water consumed) / (Total amount of liquid consumed) × 100%. A higher AI value indicates a higher degree of nausea.

The results demonstrated that both the positive control LiCl (0.15 M, 40 mL/kg, i.p.) and the GLP-1 receptor agonist hypoglycemic drugs including Liraglutide (0.1 mg/kg, i.p.), Semaglutide (0.1 mg/kg, i.p.), Semaglutide (0.1 mg/kg and 0.24 mg/kg, s.c.), and Dulaglutide (0.1 mg/kg, s.c.) induced markedly severe taste aversion. In contrast, GmFc (1.0 mg/kg and 5.0 mg/kg, s.c.) elicited no any aversion. Higher doses of GmFc (10 mg/kg and 20 mg/kg, s.c.) produced only mild aversive responses. These outcomes are presented in Figure 7, where Y-axis represents the percentage normalized aversion index to the aversion index of the positive control LiCl which was set at 100%, and the aversion index of the negative control was set at 0%. X-axis represents time post-administration in hours.

These experimental findings demonstrated that GmFc, unlike currently marketed GLP-1 receptor agonist hypoglycemic drugs Liraglutide, Semaglutide, and Dulaglutide, had no side effect like eliciting severe gastrointestinal aversion during use.

Those of ordinary skill in the art may make various improvements and supplements without departing from the spirit of the invention, which should be regarded as falling within the scope of the invention.

## Claims

1. A GLP-1 analogue-Fc fusion protein, the amino acid sequence of which is shown in SEQ ID NO. 7.

2. The GLP-1 analogue-Fc fusion protein according to claim 1, **characterized in that** the GLP-1 analogue-Fc fusion protein exists in the form of a dimer.

3. The GLP-1 analogue-Fc fusion protein according to claim 1 or 2, **characterized in that** the GLP-1 analogue-Fc fusion protein exists in the form of a homodimer.

4. A nucleic acid molecule comprising a nucleotide sequence encoding the GLP-1 analogue-Fc fusion protein according to any one of claims 1 to 3.

5. The nucleic acid molecule according to claim 4, **characterized in that** the nucleic acid molecule comprises the nucleotide sequence shown in SEQ ID NO. 8.

6. A vector comprising the nucleic acid molecule according to claim 4 or 5.

7. The vector according to claim 6, **characterized in that** the vector is an expression vector.

8. A host cell, comprising the nucleic acid molecule according to claim 4 or 5 or the vector according to claim 6 or 7, or transfected or transformed with the nucleic acid molecule according to claim 4 or 5 or the vector according to claim 6 or 7.

9. A method for producing the GLP-1 analogue-Fc fusion protein according to any one of claims 1 to 3, the method comprising expressing the vector according to claim 6 or 7 in the host cell according to claim 8.

10. Use of the GLP-1 analogue-Fc fusion protein according to any one of claims 1 to 3 in the manufacture of a medicament for treating diabetes, obesity, or overweight.

11. A pharmaceutical composition comprising the GLP-1 analogue-Fc fusion protein according to any one of claims 1 to 3 and a pharmaceutically acceptable carrier.

12. A GLP-1 analogue comprising the amino acid sequence shown in SEQ ID NO. 2.
